# FASCICULE DE BREVET EUROPEEN

(11) **EP 3 076 883 B1**
(45) Date de publication et mention de la délivrance du brevet: **17.04.2019**
(21) Numéro de dépôt: 14814949.5
(22) Date de dépôt: 24.11.2014
(51) Int. Cl.: A61B 17/32, A61B 17/34, A61B 10/02, A61B 17/3205

(54) **TROCART A BIOPSIE**
BIOPSIETROKAR
BIOPSY TROCAR

(30) Priorité: 03.12.2013 FR 1362009
(43) Date de publication de la demande: 12.10.2016
(73) Titulaire: Fumex, Laurent, Madison, CT 06443 (US); Masseglia, Thierry, 83130 La Garde (FR)
(72) Inventeur: Fumex, Laurent, Madison, CT 06443 (US); Masseglia, Thierry, 83130 La Garde (FR)
(74) Mandataire: Goulard, Sophie
(86) Numéro de dépôt international: PCT/FR2014/053014
(87) Numéro de publication internationale: WO 2015/082798

(56) Documents cités:
- US-A- 6 019 776
- US-A- 6 086 543
- US-A1- 2007 016 100
- US-A1- 2009 194 446
- US-B2- 7 850 620

## Description

La présente invention concerne un trocart destiné à la biopsie de moelle osseuse, et un dispositif destiné à la biopsie de moelle osseuse reliant un trocart à un moyen motorisé d'entrainement en rotation.

Les techniques pour réaliser les biopsies de moelle osseuse sont les biopsies par ponction et aspiration fine de la moelle (myélogramme) et les biopsies ostéo-médullaires par prélèvement de la moelle et du stroma médullaire, le choix dépendant du résultat recherché par le praticien (cytologique ou histologique).

La biopsie par aspiration peut être réalisée à l'aide d'une aiguille fine de 15-16 Gauge par exemple. En revanche, si le résultat recherché est de nature histologique, on privilégiera les trocarts ostéo-médullaires présentant un diamètre plus gros (13 Gauge par exemple) autorisant ainsi un plus gros prélèvement.

Classiquement, les biopsies de moelle osseuse type ostéo-médullaires sont surtout réalisées dans la crête de l'os iliaque présentant une fine corticale. Pour réaliser ces biopsies, les procédures pratiquées actuellement sont soit manuelles, soit motorisées. Les procédures manuelles présentent certains inconvénients dont les principaux sont liés à la pénétration dans l'os corticale et à la capture du prélèvement. La plupart des aiguilles utilisées manuellement comportent de simples mandrins à pointe trocart peu perforants, obligeant de ce fait le praticien à exercer une forte pression lors de la pénétration dans l'os. Le geste peut ainsi s'avérer particulièrement pénible à réaliser et engendrer douleur et inconfort pour le patient. Une fois la corticale pénétrée et le mandrin retiré, l'aiguille est enfoncée dans l'os de plusieurs millimètres (10 à 30 mm) afin de récupérer un prélèvement de moelle et de stroma médullaire. Avant le retrait de l'aiguille et afin de s'assurer du maintien du prélèvement des tissus dans l'aiguille, il est recommandé d'appliquer à l'aiguille un léger mouvement de bascule d'avant-arrière couplé d'un geste rotatoire. Ce geste permet de couper le spécimen et participe au succès de la capture du prélèvement ; il est cependant particulièrement douloureux pour le patient.

Les procédures motorisées atténuent significativement les difficultés de pénétration et de prélèvement et sont moins douloureuses pour le patient ; elles permettent également de réduire la durée de l'intervention pour le praticien.

Le brevet US6019776 divulgue un trocart d'accès notamment utilisé en vertébroplastie pour l'injection de matériaux, comprenant une canule et un stylet, le stylet étant guidé dans la canule. La canule et le stylet ont des filetages qui permettent à la canule d'être positionnée sur le stylet et d'avancer jusque dans une position prédéterminée. Le stylet a également un filetage pour forer l'os à son extrémité distale. Le filetage de la canule n'est pas adapté pour retenir un prélèvement de moelle car il est trop éloigné de l'extrémité distale de la canule.

Le brevet US6086543 décrit un trocart à biopsie de tissus mous. La canule du trocart comporte à sa partie distale un filetage pour retenir les tissus lors du retrait de la canule.

Le brevet US7988643 décrit un trocart à biopsie à usage manuel comportant sur l'extrémité distale de l'aiguille un filetage interne et externe ainsi qu'une partie conique. Cette aiguille est reliée à une poignée. Ce dispositif présente l'inconvénient majeur d'avoir un filetage externe qui en fonction de la densité osseuse sera très difficile à retirer sans avoir à le dévisser ce qui ne permettra pas de retirer correctement le prélèvement de moelle.

Le brevet US6110128 décrit un trocart à biopsie à usage manuel caractérisé par le fait que l'aiguille comporte des nervures internes inclinées vers l'arrière. Ce dispositif ne pourra pas effectuer des biopsies de moelle de bonne qualité car les nervures vont s'opposer à la pénétration de la moelle dans l'aiguille. En effet, le trocart étant utilisé manuellement, la vitesse de rotation n'est pas assez importante pour entraîner la moelle dans l'aiguille. En outre, la réalisation des nervures telles que décrites dans le brevet est techniquement compliquée à effectuer et très couteuse.

Le brevet US7850620 décrit un trocart à usage motorisé comportant une aiguille dont une portion de filet hélicoïdal est soudée à l'intérieur de l'extrémité distale, facilitant la montée de la moelle dans l'aiguille pendant la biopsie, et un mandrin dont l'extrémité distale est affûtée en pointe pyramidale. Ce trocart présente plusieurs inconvénients. Le diamètre externe du mandrin étant inférieur au diamètre interne du sommet de filet, il existe un jeu quelque peu significatif entre le diamètre externe du mandrin et le diamètre interne de l'aiguille. Ce jeu peut entrainer une accroche involontaire et donc des déchirures des parties molles lors de l'introduction du trocart mais aussi un bourrage de débris osseux lors de la perforation de l'os cortical. Ce bourrage peut engendrer des difficultés lors du retrait du mandrin de l'aiguille, celui-ci restant collé à la paroi interne de l'aiguille. Il peut aussi occasionner une montée involontaire de débris entre le mandrin et la paroi interne de l'aiguille, créant ainsi un tapissage de la paroi interne dont l'épaisseur peut être égale à la hauteur du sommet du filet soudé. L'efficacité du système de capture du spécimen, et donc du filet soudé, est alors remise en cause car le filet soudé se trouve en effet noyé dans l'épaisseur du tapissage et devient inopérant pour l'étape suivante de la réalisation de la biopsie proprement dite. Enfin, la réalisation d'un tel filet par soudure est techniquement difficile et est particulièrement couteuse. La difficulté s'accroit naturellement de manière significative en cas de diminution de la taille des aiguilles.

Ces trocarts permettent effectivement la réalisation de biopsie de moelle avec des techniques opératoires plus longues et douloureuses pour les dispositifs manuels et des risques d'échecs dus au bourrage pour le dispositif motorisé.

La présente invention a pour objet de fournir un trocart destiné à réaliser des biopsies de moelle osseuse permettant de réaliser des biopsies fiables, sûres et rapides quel que soit le diamètre de l'aiguille, et pour un prix de revient raisonnable.

Le trocart à biopsie selon la présente invention est défini dans la revendication 1 et comprend une aiguille à biopsie comprenant une canule et un mandrin comprenant un axe, l'axe étant adapté pour coulisser dans l'aiguille à biopsie. La canule comporte à son extrémité distale au moins une nervure interne formant une portion d'hélice sur la paroi interne de la canule, et l'axe comporte au moins une rainure hélicoïdale coopérant avec la nervure interne. La nervure interne est adaptée pour retenir un prélèvement de moelle dans l'aiguille.

L'axe de la portion d'hélice de la nervure interne est décentré par rapport à l'axe de la canule. Ainsi, la largeur ou l'épaisseur de la nervure interne, dans le plan de la section transversale de la canule, varie de 0 à ses extrémités à quelques dixièmes du diamètre interne de la canule au centre de la nervure interne. Ces caractéristiques différencient la nervure interne d'un filetage interne.

Grâce à la nervure, le prélèvement de moelle est bien retenu dans l'aiguille lorsque celle-ci est retirée du patient. Aussi, la faible étendue (ou longueur) de la nervure interne sur le diamètre interne de la canule n'empêche pas la moelle de pénétrer dans l'aiguille mais le facilite, tout en exerçant peu d'effort de rétention permettant une éjection aisée du prélèvement de moelle sans détérioration.

De manière avantageuse, la canule ne comporte qu'une seule nervure interne. De cette façon, la pénétration de la moelle dans la canule est considérablement facilitée.

Selon des modes de réalisation, la largeur de la nervure interne varie de 0 à ses extrémités à 20% de la valeur du diamètre interne de la canule au centre.

De manière avantageuse, l'inclinaison de la nervure interne par rapport à l'axe de la canule et l'inclinaison de la rainure hélicoïdale sont identiques.

Selon des modes de réalisation, l'inclinaison de la nervure interne par rapport à l'axe de la canule est d'une valeur comprise entre 65° et 80°.

De préférence, l'inclinaison de la nervure interne par rapport à l'axe de la canule est de 75°.

Suivant une caractéristique de l'invention, la nervure interne s'étend sur la paroi interne de la canule selon un angle compris entre 30° et 180°.

Avantageusement, la nervure interne a une section transversale de forme angulaire avec un angle compris entre 10° et 120°.

De manière préférentielle, l'angle de la section transversale est de 60°.

Préférentiellement, la nervure interne est une portion d'hélice dont le pas est à droite et la rainure hélicoïdale a un pas à droite.

Avantageusement, le mandrin se visse dans l'aiguille. L'ensemble formé de l'aiguille et du mandrin est adapté pour être monté dans un moyen automatique d'entrainement en rotation.

Selon un autre aspect, la présente invention porte également sur un dispositif à biopsie destiné à réaliser des biopsies de moelle osseuse, comprenant un trocart selon des modes de réalisation décrits ci-dessus et un moyen automatique d'entrainement en rotation auquel le trocart est solidarisé. Ce moyen automatique d'entrainement en rotation peut être une perceuse, réalisant, par exemple, 300 à 400 tours par minute.

Grâce à la nervure interne, le prélèvement de moelle est entrainé plus facilement dans la canule, le dispositif s'apparentant à celui d'une vis sans fin de par la combinaison de l'inclinaison de la nervure interne et de la vitesse de rotation de la perceuse. En effet, comme la vitesse de pénétration du dispositif dans l'os ou dans la moelle est toujours plus faible que la vitesse de pénétration théorique de la nervure interne, du fait de son pas théorique, la montée du prélèvement dans l'aiguille est garantie.

La présence d'une nervure interne et d'une rainure hélicoïdale qui s'emboitent l'une dans l'autre permet également d'ajuster au plus près le mandrin et l'aiguille et de réduire ainsi les risques d'accroche des parties molles puis de bourrage par des débris osseux lors de la pénétration dans la corticale. Une fois le mandrin retiré, l'aiguille est bien vide de tout débris et la ou les nervures prêtes à jouer pleinement leur rôle.

La canule de l'aiguille à biopsie peut être collée ou surmoulée dans un corps et l'axe du mandrin peut être collé ou surmoulé dans un bouchon. La canule comporte à son extrémité distale un affûtage à facettes couramment utilisé pour ce type de biopsie. L'axe est affûté à son extrémité distale de façon à forer l'os. La pointe peut être triangulaire, lancéolée ou une mèche.

Avantageusement, la canule et l'axe sont réalisés dans des matériaux biocompatibles, par exemple de type aciers inoxydables. Le corps et le bouchon sont en plastique à usage médical.

Les avantages de la présente invention seront plus clairs avec l'explication de pose suivante. Le praticien saisit le mandrin, le glisse dans l'aiguille à biopsie et solidarise l'ensemble en vissant le mandrin dans l'aiguille à biopsie jusqu'au blocage du bouchon contre le corps. Cet ensemble forme le trocart. Le praticien encliquette le trocart dans l'embout spécialement adapté d'une perceuse stérile, puis traverse les tissus mous jusqu'au contact osseux. A ce moment, il active la perceuse et fore l'os cortical. L'os cortical étant perforé, il décliquette le trocart de l'embout de la perceuse, dévisse le mandrin et le retire de l'aiguille à biopsie figée dans l'os. Il encliquette de nouveau la perceuse sur l'aiguille à biopsie puis prélève la moelle osseuse en actionnant et en poussant la perceuse. Quand la profondeur désirée est atteinte, après avoir laissé tourner la perceuse quelques secondes à la même position afin d'assurer le sectionnement de la moelle, il retire la perceuse avec l'aiguille tout en laissant la perceuse actionnée. Après avoir décliqueté la perceuse, le praticien éjecte le prélèvement de l'aiguille à biopsie en le poussant par l'extrémité distale de l'aiguille au moyen d'un éjecteur.

D'autres caractéristiques et avantages de la présente invention apparaîtront dans la description suivante, relative à des modes préférentiels de réalisation de l'invention, en référence aux dessins annexés, qui représentent :
Figure 1 : vue en perspective d'un trocart selon l'invention ;
Figure 2 : vue en perspective de l'extrémité distale du trocart selon l'invention ;
Figure 3 : vue d'une aiguille à biopsie du trocart selon l'invention ;
Figures 4 à 6 : vues illustrant l'extrémité distale d'une canule du trocart selon l'invention ;
Figure 7 : vue d'un mandrin du trocart selon l'invention ;
Figure 8 : vues de l'extrémité d'un axe du trocart selon l'invention ;
Figure 9 : vue en coupe de l'extrémité distale du trocart selon l'invention ;
Figure 10 : vue en coupe de l'extrémité distale d'une variante du trocart selon l'invention ;
Figures 11 à 12: vues de l'extrémité distale d'une variante de la canule selon l'invention ; et
Figure 13 : vue de l'extrémité distale d'une variante de l'axe selon l'invention.

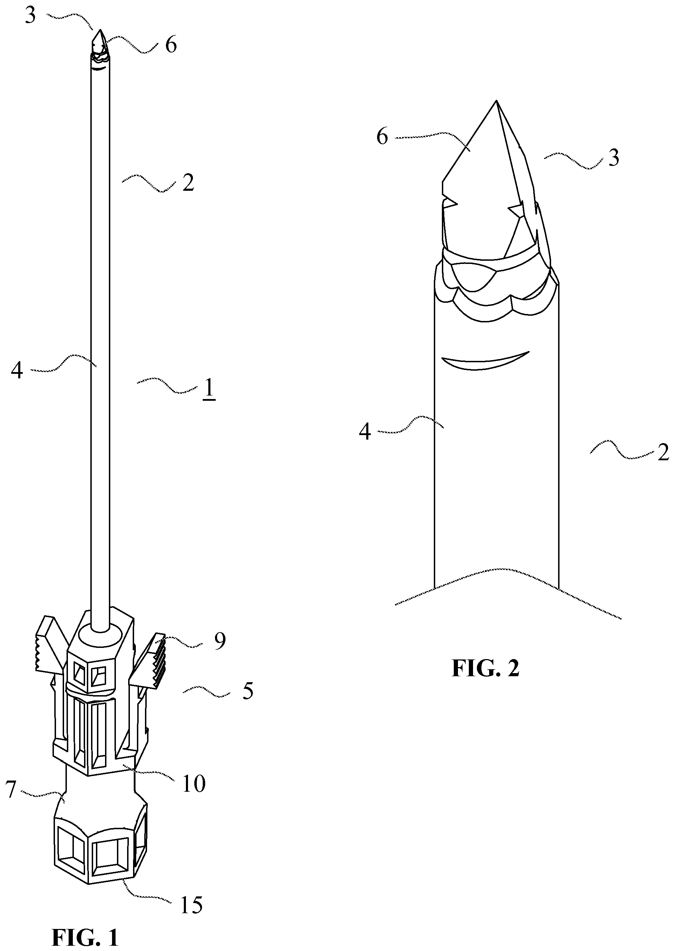

Le trocart 1 selon la présente invention, représentée sur la Figure 1, est composé d'une aiguille à biopsie 2 et d'un mandrin 3. L'aiguille à biopsie 2 est constituée d'une canule 4 logé dans un corps 5. Le mandrin 3 est constitué d'un axe 6 logé dans un bouchon 7. La Figure 2 montrant l'extrémité distale du trocart 1 plus en détails.

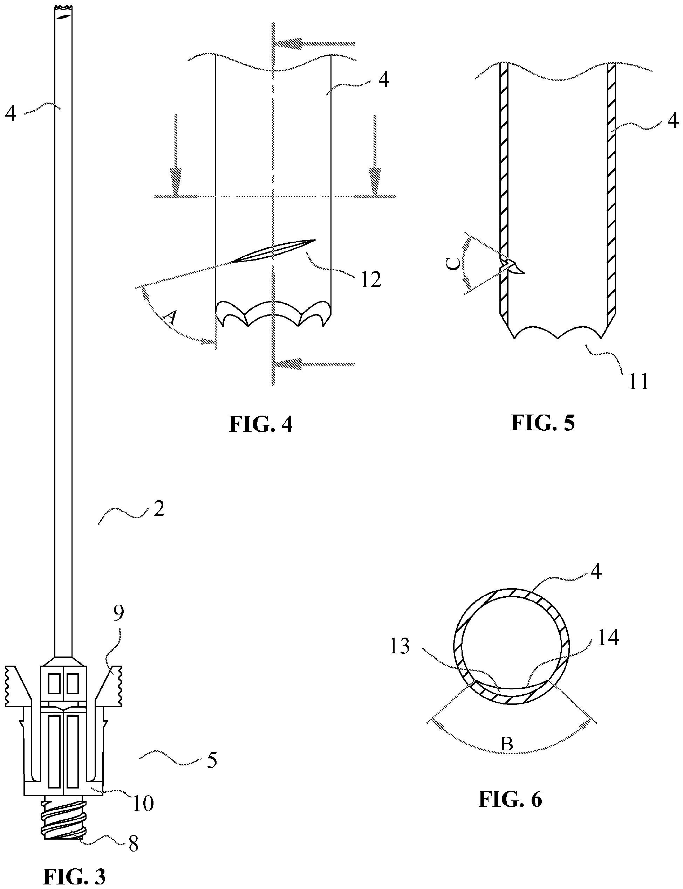

La Figure 3 représente l'aiguille à biopsie 2 dont le corps 5 est composé d'un embout Luer 8 et d'un système d'encliquetage 9. La forme 10 du corps 5 est hexagonale coopérant, par exemple, avec une cavité hexagonale d'un embout d'une perceuse. Le système d'encliquetage 9 comporte deux parties flexibles s'encliquetant dans des évidements de l'embout de la perceuse, permettant le maintien et l'entrainement en rotation du trocart 1. L'embout Luer 8 permet de raccorder, par exemple, une seringue à l'aiguille à biopsie 2 pour réaliser une aspiration de moelle si cela est nécessaire lors d'un acte chirurgical.

Les figures 4 à 6 représentent la partie distale de la canule 4. Les Figures 5 et 6 montrent des coupes selon les lignes indiquées dans la Figure 4. Un affûtage à biseaux 11 pratiqué à l'extrémité distale de la canule 4 est traditionnellement utilisé pour la biopsie de moelle osseuse. La canule 4 comporte en outre une nervure interne 12. La nervure interne 12 est orientée de façon à former une portion d'hélice sur la paroi intérieure de la canule 4. Le pas de l'hélice est à droite car le sens de rotation manuelle ou automatique du trocart lors de son opération est à droite. La portion d'hélice est inclinée selon un angle A par rapport à l'axe de la canule 4. Selon des modes de réalisation, la valeur de l'angle A est comprise entre 65° et 80°, et est de préférence de 75°. La nervure interne 12 est réalisée, par exemple, à environ 2 mm de l'extrémité distale de la canule 4. Comme illustré sur la Figure 6, la nervure interne 12 forme un arc de cercle 13 ayant un angle B compris entre 30° et 180°. Préférentiellement, l'angle B de l'arc de cercle 13 est d'environ 120°.

De préférence, la nervure interne 12 a une section transversale de forme angulaire, comme montré, par exemple, sur la Figure 5. L'angle C a une valeur comprise entre 10° et 120°. De préférence, la valeur de l'angle C est de 60°.

La nervure interne 12 est de préférence réalisée par un emboutissage de la paroi externe de la canule 4, comme illustré sur les Figures 4 et 5. Cet emboutissage, réalisé au moyen d'une matrice cylindrique comportant au moins une rainure hélicoïdale correspondant à la forme à obtenir qui est logé dans la canule 4 et d'au moins un poinçon, dont l'extrémité distale correspond à la forme à obtenir, traversant un bloc de métal dans lequel la canule 4 est logée avec la matrice dans un alésage dont le diamètre est légèrement supérieur au diamètre externe de la canule 4, permet d'obtenir la ou les nervures sans déformation et perforation du diamètre externe de la canule 4. Ceci permet la fabrication rapide et simple de la nervure interne 12 en évitant des travaux de soudure dans la canule 4, celle-ci n'ayant que quelques mm de diamètre interne. En outre, l'emboutissage peut être pratiqué sur toutes sortes de trocarts existants, les rendant ainsi plus performants.

En se référant à la Figure 6, la valeur du diamètre du sommet 14 de l'hélice de la nervure interne 12 est supérieure à la valeur du diamètre interne de la canule 4. L'axe de la portion d'hélice du sommet 14 de la nervure interne 12 est décentré par rapport à l'axe de la canule. Ainsi, la largeur ou l'épaisseur de la nervure interne 12, dans le plan de la section transversale de la canule 4, varie de 0 mm à ses extrémités à quelques dixièmes par rapport au diamètre interne de la canule 4 au centre de la nervure interne 12. Par exemple, la largeur maximale de la nervure interne 12 au centre peut être comprise entre 0,1 mm et 0,5 mm pour un diamètre interne de la canule 4 de 2,5 mm, ce qui correspond à une largeur maximale d'environ 20% de la valeur du diamètre interne de la canule 4.

Bien entendu, la représentation de la forme du sommet 14 de la nervure 12 ne se limite pas à une seule forme cylindrique mais peut être de forme plane ou être une combinaison de formes planes et ou une combinaison de formes planes et cylindriques. Dans tous les cas, les parties formant le sommet 14 de la nervure 12 peuvent être considérées comme des portions d'hélice à diamètre variable. L'axe d'au moins une des parties du sommet 14 est alors décentré par rapport à l'axe de la canule 4.

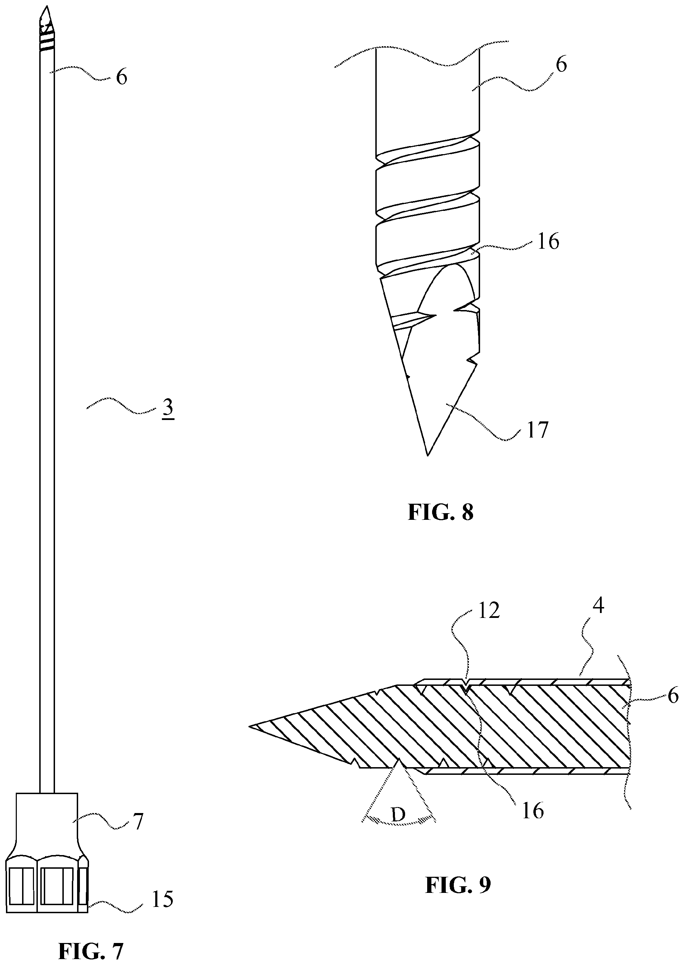

La Figure 7 représente le mandrin 3 dont le bouchon 7 a une forme hexagonale 15 coopérant avec la cavité de forme hexagonale de l'embout d'une perceuse, permettant le maintien en position du mandrin 3 par rapport à l'aiguille à biopsie 2 pendant la rotation de la perceuse.

La Figure 8 représente la partie distale de l'axe 6 comportant une rainure hélicoïdale 16 dont l'angulation coopère avec l'angle A de la nervure interne 12 de la canule 4. La section transversale de la rainure hélicoïdale 16 a une forme angulaire. L'extrémité distale de l'axe 6 comporte une partie coupante 17 affûtée de façon traditionnelle permettant le forage de l'os cortical iliaque.

La Figure 9 représente une vue en coupe longitudinale de l'extrémité distale du trocart 1 comprenant la canule 4 et l'axe 6. On peut apercevoir le jeu existant entre la nervure interne 12 et la rainure hélicoïdale 16. L'angle D de la rainure hélicoïdale 16 est supérieur ou égal à l'angle C de la nervure interne 12 (voir Figure 5) et la profondeur de la rainure hélicoïdale 16 est supérieure à la largeur ou l'épaisseur maximale de la nervure interne 12.

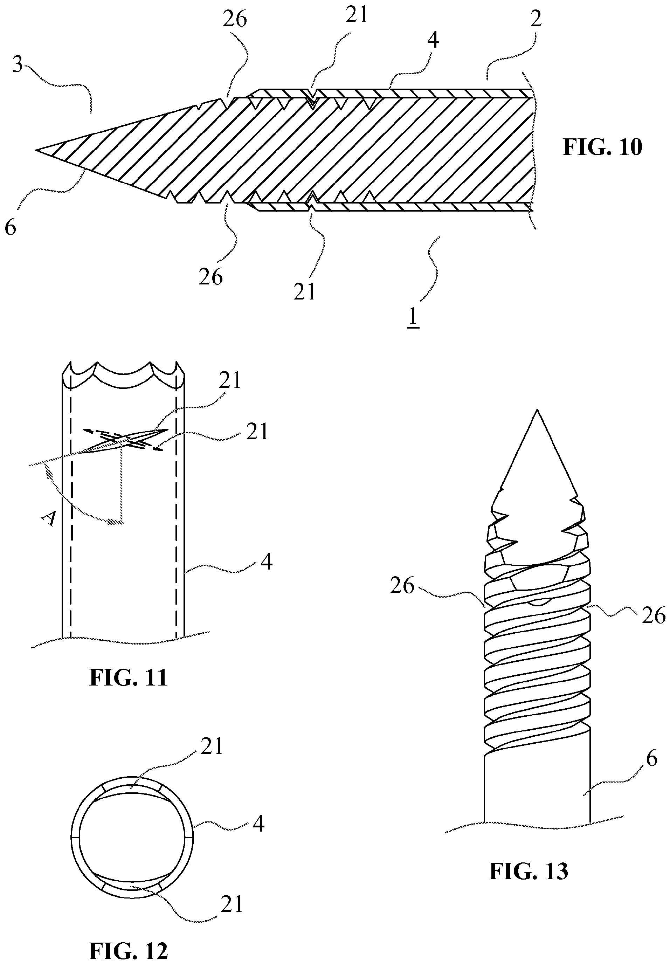

La Figure 10 représente une vue en coupe de l'extrémité distale du trocart 1 selon une variante. Le trocart 1 comprend une aiguille à biopsie 2 dont la canule 4 comporte deux nervures internes 21 diamétralement opposées et un mandrin 3 dont l'axe 6 comporte deux rainures hélicoïdales 26 coopérant avec les nervures internes 21 de la canule 4.

Les Figures 11 et 12 représentent des vues de la partie distale de la canule 4 comportant à son extrémité distale deux nervures internes 21 diamétralement opposées. Les nervures internes 21 peuvent être réalisées à environ 2 mm de l'extrémité distale de la canule 4. Elles sont orientées de façon à former deux portions d'hélices dont le pas est à droite et sont inclinées par rapport à l'axe de la canule 4 selon le même angle A.

La Figure 13 représente la partie distale de l'axe 6 comprenant deux rainures hélicoïdales 26 dont les angulations coopèrent avec l'angle A de des nervures internes 21.

Le dispositif à biopsie selon la présente invention, destiné à réaliser des biopsies de moelle osseuse, comprend un trocart 1 selon l'un des modes de réalisation décrits ci-dessus et une perceuse (non représentée) sur laquelle le trocart 1 est monté. Selon un exemple, la perceuse ayant une vitesse de rotation de 6 tours/s, pour une profondeur de moelle osseuse à pénétrer de 30 mm et le temps moyen de réalisation du prélèvement étant de 4 s, la vitesse moyenne de pénétration de la canule est de 7,5 mm/s. Le pas théorique de l'hélice de la nervure interne 12 étant de 1,39 mm, la vitesse théorique de pénétration de la canule 4 est de 1,39 mm x 6 tours/s = 8,34 mm/s. La différence entre la vitesse moyenne de pénétration et la vitesse théorique de pénétration de la canule 4 génère donc un effet de vis sans fin grâce auquel la moelle osseuse est entrainée dans la canule 4.

## Revendications

1. Trocart (1) à biopsie destiné à réaliser des biopsies de moelle osseuse, comprenant :
- une aiguille à biopsie (2) comprenant une canule (4), et
- un mandrin (3) comprenant un axe (6), l'axe (6) étant adapté pour coulisser dans l'aiguille à biopsie (2);
la canule (4) comportant à son extrémité distale au moins une nervure interne (12, 21) formant une portion d'hélice sur la paroi interne de la canule (4), la nervure interne (12, 21) étant adaptée pour retenir un prélèvement de moelle dans l'aiguille (2); **caractérisé en ce que** :
- l'axe de la portion d'hélice d'au moins une partie du sommet (14) de la nervure interne (12, 21) est décentré par rapport à l'axe de la canule (4) et le diamètre dudit sommet a une valeur supérieure à la valeur du diamètre interne de la canule,
- l'axe (6) du mandrin comporte au moins une rainure hélicoïdale (16, 26) coopérant avec la nervure interne (12, 21).

2. Trocart (1) selon la revendication 1, dans lequel la largeur de la nervure interne (12, 21) varie de 0 à ses extrémités à quelques dixièmes de la valeur du diamètre interne de la canule (4) au centre.

3. Trocart (1) selon l'une des revendications 1 à 2, dans lequel l'inclinaison de la nervure interne (12, 21) par rapport à l'axe de la canule (4) et l'inclinaison de la rainure hélicoïdale (16, 26) sont identiques.

4. Trocart (1) selon l'une des revendications précédentes, dans lequel l'inclinaison de la nervure interne (12, 21) par rapport à l'axe de la canule (4) est d'une valeur comprise entre 65° et 80°.

5. Trocart (1) selon l'une des revendications précédentes, dans lequel l'inclinaison de la nervure interne (12, 21) par rapport à l'axe de la canule (4) est de 75°.

6. Trocart (1) selon l'une des revendications précédentes, dans lequel la nervure interne (12, 21) s'étend sur la paroi interne de la canule (4) selon un angle compris entre 30° et 180°.

7. Trocart (1) selon l'une des revendications précédentes, dans lequel la nervure interne (12, 21) a une section transversale de forme angulaire avec un angle compris entre 10° et 120°.

8. Trocart (1) selon la revendication 7, dans lequel l'angle de la section transversale est de 60°.

9. Trocart (1) selon l'une des revendications précédentes, dans lequel la nervure interne (12, 21) est une portion d'hélice dont le pas est à droite et la rainure hélicoïdale (16, 26) a un pas à droite.

10. Trocart (1) selon l'une des revendications précédentes, dans lequel le mandrin (3) se visse dans l'aiguille (2).

11. Trocart (1) selon l'une des revendications précédentes, dans lequel l'ensemble formé de l'aiguille (2) et du mandrin (3) est adapté pour être monté dans un moyen automatique d'entrainement en rotation.

12. Trocart (1) selon l'une des revendications précédentes, dans lequel la nervure interne (12) est réalisée par emboutissage de la paroi externe de la canule (4).

13. Dispositif à biopsie destiné à réaliser des biopsies de moelle osseuse, comprenant un trocart (1) selon l'une des revendications précédentes et un moyen automatique d'entrainement en rotation auquel le trocart (1) est solidarisé.

## Patentansprüche

1. Biopsietrokar (1) für die Durchführung von Knochenmarksbiopsien, umfassend:
- eine Biopsienadel (2), umfassend eine Kanüle (4), und
- einen Dorn (3), umfassend eine Achse (6), wobei die Achse (6) zum Gleiten in der Biopsienadel (2) geeignet ist,
wobei die Kanüle (4) an ihrem distalen Ende mindestens eine interne Rille (12, 21) aufweist, die einen Wendelabschnitt auf der inneren Wand der Kanüle (4) bildet, wobei die interne Rille (12, 21) zum Zurückhalten einer Markprobe in der Nadel (2) geeignet ist,
**dadurch gekennzeichnet, dass**:
- die Achse (6) des Wendelabschnitts von mindestens einen Teil der Spitze (14) der internen Rille (12, 21) in Bezug auf die Achse der Kanüle (4) dezentriert ist und der Durchmesser der Spitze einen Wert über dem Wert des inneren Durchmessers der Kanüle hat,
- die Achse (6) des Dorns mindestens eine schraubenförmige Rille (16, 26) hat, die mit der internen Rille (12, 21) zusammenwirkt.

2. Trokar (1) nach Anspruch 1 oder 2, wobei die Breite der internen Rille (12, 21) von 0 an ihren Enden bis einige Zehntel des Wertes des Innendurchmessers der Kanüle (4) im Zentrum schwankt.

3. Trokar (1) nach einem der Ansprüche 1 bis 2, wobei die Neigung der internen Rille (12, 21) in Bezug auf die Achse der Kanüle (4) und die Neigung der schraubenförmigen Rille (16, 26) identisch sind.

4. Trokar (1) nach einem der vorangehenden Ansprüche, wobei die Neigung der internen Rille (12, 21) in Bezug auf die Achse der Kanüle (4) bei einem Wert zwischen 65° und 80° liegt.

5. Trokar (1) nach einem der vorangehenden Ansprüche, wobei die Neigung der internen Rille (12, 21) in Bezug auf die Achse der Kanüle (4) 75° beträgt.

6. Trokar (1) nach einem der vorangehenden Ansprüche, wobei sich die interne Rille (12, 21) auf der inneren Wand der Kanüle (4) gemäß einem Winkel erstreckt, der zwischen 30° und 180° liegt.

7. Trokar (1) nach einem der vorangehenden Ansprüche, wobei die interne Rille (12, 21) einen winkelförmigen Querschnitt mit einem Winkel hat, der zwischen 10° und 120° liegt.

8. Trokar (1) nach Anspruch 8, wobei der Winkel des Querschnitts 60° beträgt.

9. Trokar (1) nach einem der vorangehenden Ansprüche, wobei die interne Rille (12, 21) ein Wendelabschnitt ist, dessen Steigung nach rechts ist und die schraubenförmige Rille (16, 26) eine Steigung nach rechts hat.

10. Trokar (1) nach einem der vorangehenden Ansprüche, wobei der Dorn (3) in die Nadel (2) geschraubt wird.

11. Trokar (1) nach einem der vorangehenden Ansprüche, wobei die Einheit, gebildet von der Nadel (2) und dem Dorn (3) für das Anbringen in einem automatischen Rotationsantriebsmittel geeignet ist.

12. Trokar (1) nach einem der vorangehenden Ansprüche, wobei die interne Rille (12) durch Tiefziehen der Außenwand der Kanüle (4) hergestellt ist.

13. Biopsievorrichtung, die zur Durchführung von Knochenmarksbiopsie bestimmt ist, umfassend einen Trokar (1) nach einem der vorangehenden Ansprüche und ein automatisches Rotationsantriebsmittel, mit dem der Trokar (1) fest verbunden ist.

## Claims

1. A biopsy trocar (1) intended to perform bone marrow biopsies, comprising:
- a biopsy needle (2) comprising a cannula (4), and
- a mandrel (3) comprising an axle (6), the axle (6) being suitable for sliding in the biopsy needle (2);
the cannula (4) including, at its distal end, at least one inner rib (12, 21) forming a helix portion on the inner wall of the cannula (4), the inner rib (12, 21) being suitable for holding a marrow specimen in the needle (2);
**characterized in that**
- the axis of the helix portion of at least part of the apex (14) of the inner rib (12, 21) is off-centered relative to the axis of the cannula (4) and the diameter of said apex has a value greater than the value of the inner diameter of the cannula,
- the axis (6) of the mandrel includes at least one helical groove (16, 26) cooperating with the inner rib (12, 21).

2. The trocar (1) according to claim 1, wherein the width of the inner rib (12, 21) varies from 0 at its ends to several tenths of the value of the inner diameter of the cannula (4) at the center.

3. The trocar (1) according to one of claims 1 to 2, wherein the incline of the inner rib (12, 21) relative to the axis of the cannula (4) and the incline of the helical groove (16, 26) are identical.

4. The trocar (1) according to one of the preceding claims, wherein the incline of the inner rib (12, 21) relative to the axis of the cannula (4) has a value of between 65° and 80°.

5. The trocar (1) according to one of the preceding claims, wherein the incline of the inner rib (12, 21) relative to the axis of the cannula (4) is 75°.

6. The trocar (1) according to one of the preceding claims, wherein the inner rib (12, 21) extends over the inner wall of the cannula (4) along an angle of between 30° and 180°.

7. The trocar (1) according to one of the preceding claims, wherein the inner rib (12, 21) has an angular cross-section with an angle of between 10° and 120°.

8. The trocar (1) wherein the angle of the cross-section is 60°.

9. The trocar (1) according to one of the preceding claims, wherein the inner rib (12, 21) is a helix portion with a right pitch and the helical groove (16, 26) has a right pitch.

10. The trocar (1) according to one of the preceding claims, wherein the mandrel (3) screws into the needle (2).

11. The trocar (1) according to one of the preceding claims, wherein the assembly made up of the needle (2) and the mandrel (3) is suitable for being mounted in an automated rotational drive means.

12. The trocar (1) according to one of the preceding claims, wherein the inner rib (12) is made by stamping the outer wall of the cannula (4).

13. A biopsy device intended to perform bone marrow biopsies, comprising a trocar (1) according to one of the preceding claims and an automatic rotational drive means to which the trocar (1) is secured.
